# EUROPEAN PATENT APPLICATION

(11) **EP 2 357 223 A2**
(43) Date of publication of application: **17.08.2011**
(21) Application number: 11152200.9
(22) Date of filing: 07.11.2003
(51) Int. Cl.: C12N 5/00, C12N 5/02, A61K 35/26, A61K 35/28

(54) **Human stem cell material and methods**

(30) Priority: 07.11.2002 US 424442 P
(62) Divisional of application: 03781813.5
(71) Applicant: UNIVERSITY OF CHICAGO, Chicago, IL 60637 (US)
(72) Inventor: Zhao, Yong, Lisle, IL 60532 (US)
(74) Representative: Witte, Weller & Partner

(57) **Abstract**

Monocyte derived adult stem cells (MDSCs) isolated from peripheral blood of mammals is provided, along with pharmaceutical compositions containing an MDSC, kits containing a pharmaceutical composition, and methods of preparing, propagating and using MDSCs or differentiated derivatives thereof. The uses of these biological materials include methods of treating disorders or diseases, as well as methods of ameliorating a symptom associated with any such disorder or disease.

## Description

This application claims the benefit of U.S. Provisional Application No. 60/424,442, filed November 7, 2002, which is incorporated herein by reference in its entirety.

### GOVERNMENT INTEREST

The U.S. government owns rights in the invention pursuant to National Cancer Institute grant number 1 R01 CA 80826-01.

### TECHNICAL FIELD

The invention generally relates to methods of isolating, culturing, propagating, and differentiating adult stem cells derived from a subset of cultured peripheral blood monocytes.

### BACKGROUND

Pluripotent stem cells are a valuable resource for research, drug discovery and therapeutic treatments, including transplantation (Lovell-Badge, Nature, 414:88-91 (2001); Donovan et al., Nature, 414, 92-97 (2001); Griffith et al., Science, 295:1009-1014 (2002); Weissman, N. Engl. J. Med., 346:1576-1579 (2002)). These cells, or their mature progeny, can be used to study signaling events that regulate differentiation processes, identify and test drags for lineage-specific beneficial or cytotoxic effects, or replace tissues damaged by disease or an environmental impact. The current state of pluripotent stem cell biology and the medicinal outlook, however, are not without drawbacks or free from controversy.

The use of pluripotent stem cells from fetuses, umbilical cords or embryonic tissues derived from *in vitro* fertilized eggs raises ethical and legal questions in the case of human materials, poses a risk of transmitting infections and/or may be ineffective because of immune rejection. In particular, embryonic stem cells have a number of disadvantages. For example, embryonic stem cells may pass through several intermediate stages before becoming the cell type needed to treat a particular disease. In addition, embryonic stem cells may be rejected by the recipient's immune system since it is possible that the immune profile of the specialized cells would differ from that of the recipient.

One way to circumvent these problems is by exploiting autologous stem cells, preferably from an easily accessible tissue such as peripheral blood. In this context, it has been reported that bone marrow contains cells that appear to have the ability to trans-differentiate into mature cells belonging to cell lineages other than those of the blood (Laggase et al., Nature Med., 6:1229-1234 (2000); Orlic et al., Nature, 410:640-641 (2001); Korbling, et al. N. Engl. J. Med., 346:738-746 (2002)). However, recent studies have questioned the existence of such a trans-differentiation and raised the possibility that the emerging mature cells result from fusion of stem cells with resident tissue cells (Terada, et al., Nature, 416,:542-545 (2002); Ying et al., Nature, 416:545-548 (2002)). A further consideration is that obtaining samples from bone marrow is often a painful and cumbersome procedure.

In a separate study, Jiang et al. observed that a cell within mesenchymal cell cultures derived from the bone marrow of rats, mice and humans had the ability to differentiate into various cell lineages (Jiang et al., Nature 418:41-49 (2002)). Again, however, these cells are located in the relatively inaccessible bone marrow of these rodents, making their isolation and use a more difficult and costly process.

Thus, needs exist in the art to isolate, culture, sustain, propagate, and differentiate adult stem cells, particularly human adult stem cells that are relatively accessible in order to develop cell types suitable for a variety of uses. Such uses may include the use of autologous stem cells for the treatment of diseases and amelioration of symptoms of diseases.

### SUMMARY OF THE INVENTION

The invention solves the aforementioned need(s) in the art by generally providing a monocyte-derived stem cell (MDSC) that is pluripotent, along with pharmaceutical compositions including such a cell, methods of preparing and sustaining such a cell, methods of propagating such a cell, methods of differentiating such a cell, methods of propagating a non-terminally differentiated cell, and methods of using a cell or cells from the group comprising a MDSC and differentiated cells thereof to treat diseases or disorders or to ameliorate symptoms associated with a disease or disorder. The MDSCs of the invention are found in peripheral blood, providing a cost-effective source of pluripotent stem cells that can be obtained from most organisms. Significantly, these MDSCs can be readily propagated. Because such cells are typically available from most organisms, autologous MDSCs are also available where necessary or desired. Moreover, as pluripotent stem cells, the MDSCs of the invention are suitable for use in treating a wide variety of disorders and diseases, and in ameliorating a symptom associated with one or more of those diseases or disorders.

In one aspect, the invention provides a method of preparing an isolated monocyte-derived stem cell (MDSC) comprising the steps of isolating a peripheral-blood monocyte (PBM); contacting the PBM with an effective amount of a mitogenic compound selected from the group consisting of macrophage colony-stimulating factor (M-CSF), interleukin-6 (IL-6) and leukemia inhibitory factor (LIF); and culturing the PBM under conditions suitable for propagation of the cell and thereby obtaining a preparation of an isolated MDSC. The PBM is preferably a mammalian, human, or adult human PBM. In one embodiment, the PBM is cryopreserved prior to contact with a mitogenic compound. In a related aspect of the invention, the isolated MDSC is cryopreserved.

In another related aspect, the invention comprehends an isolated MDSC obtained by the above-described method. As the MDSC of the invention has a distinct phenotype, it is contemplated by the invention that the MDSC will have at least one specific and characteristic activity. For example, an MDSC of the invention exhibits at least one distinct cell surface marker (MAC-1, CD14, CD34, CD40 and CD45), or produces at least one cytokine selected from the group consisting of IL-1β, IL-6 and IL-12 p70, or exhibits phagocytic activity, or exhibits lymphocyte activation activity, or exhibits resistance to dispersion by any one of trypsin, EDTA and dispase, or exhibits susceptibility to dispersion by lidocaine. Preferably an isolated MDSC according to the invention exhibits phagocytic activity. Also preferred is an isolated MDSC exhibiting at least one of the above-identified cell surface markers, production of one of the above-identified cytokines, phagocytic activity, lymphocyte activation activity, resistance to dispersion by trypsin, EDTA, or dispase, and susceptibility to dispersion by lidocaine.

Isolated MDSCs exhibiting a variety of cell-surface antigens are contemplated in the invention. In one aspect of the invention, an isolated MDSC is provided wherein the cell exhibits a surface antigen selected from the group consisting of MAC-1, CD14, CD34, CD40 and CD45. Preferably, the invention provides an isolated MDSC wherein the MDSC does not exhibit a surface antigen selected from the group consisting of CD1a and CD83.

In one embodiment of this aspect of the invention, an isolated MDSC is provided wherein the cell produces a cytokine selected from the group consisting of IL-1ß, IL-6 and IL-12 p70. In another embodiment, the invention provides an isolated MDSC that exhibits phagocytic activity.

In another embodiment, the MDSC of the invention is resistant to dispersion by an agent selected from the group consisting of trypsin, EDTA and dispase. In yet another embodiment, the MDSC of the invention is susceptible to dispersion following treatment with lidocaine. Of course, an MDSC according to the invention may be resistant to dispersion by trypsin, EDTA and dispase, while being susceptible to dispersion with lidocaine.

The invention also comprehends an isolated MDSC wherein the cell is an adult human cell; exhibits a surface antigen selected from the group consisting of MAC-1, CD14, CD34, CD40 and C45; produces a cytokine selected from the group consisting ofIL-1B, IL-6 and IL-12 p70; is resistant to dispersion by an agent selected from the group consisting of trypsin, EDTA, and dispase; and exhibits phagocytic activity.

In another aspect of the invention, a method of generating a differentiated cell is provided comprising the steps of isolating an MDSC and contacting the cell with an amount of an inducing agent effective to induce differentiation of the cell. Preferably, the differentiated cell is cultured under conditions for sustaining and/or propagating the cell. The MDSC of the invention is preferably a human MDSC or an adult human MDSC. In a related aspect, the invention contemplates cryopreservation of the MDSC and/or the differentiated cell.

A related aspect of the invention provides a method for identifying a cell type-specific therapeutic agent comprising contacting a candidate therapeutic agent and a first differentiated cell obtained according to the above-described method of generating a differentiated cell, further contacting the candidate therapeutic agent and a second differentiated cell obtained according to that method of generating a differentiated cell, wherein the first and second differentiated cells are different cell types, and measuring the viability of the first differentiated cell relative to the viability of the second differentiated cell, wherein a difference in viabilities identifies the candidate therapeutic agent as a cell type-specific therapeutic agent.

Given the scope of the invention, one skilled in the art will appreciate that a variety of growth and differentiation factors and methods, which are employed in the generation, sustaining and/or propagation of a range of specific cell and tissue types, can be used in sustaining, propagation and/or differentiation of the MDSC described herein. In particular, the invention contemplates a method of generating, sustaining and/or propagating a neuronal cell comprising the steps of isolating an MDSC; contacting the MDSC with an amount of a nerve cell inducing agent such as nerve growth factor (bNGF) effective to induce MDSC differentiation into a neuronal cell; and culturing the neuronal cell under conditions suitable for sustaining and/or propagating the neuronal cell.

In another aspect of the invention, a method of generating, sustaining and/or propagating an endothelial cell is provided comprising the steps of isolating an MDSC; contacting the MDSC with an amount of an endothelial cell inducing agent such as vascular endothelial growth factor (VEGF) effective to induce MDSC differentiation into an endothelial cell; and culturing the endothelial cell under conditions suitable for sustaining and/or propagating the endothelial cell.

In another aspect of the invention, a method of generating, sustaining and/or propagating an epithelial cell is provided comprising the steps of isolating an MDSC; contacting the MDSC with an amount of an epidermal cell inducing agent such as epidermal growth factor (EGF) effective to induce MDSC differentiation into an epithelial cell; and culturing the epithelial cell under conditions suitable for sustaining and/or propagating the epithelial cell.

In yet another aspect of the invention, a method of generating, sustaining and/or propagating a T-lymphocyte is provided comprising the steps of isolating an MDSC; contacting the MDSC with an amount of a T-cell inducing agent such as interleukin-2 (IL-2) effective to induce MDSC differentiation into a T-lymphocyte; and culturing the T-lymphocyte under conditions suitable for sustaining and/or propagating the T-lymphocyte.

In still another aspect of the invention, a method of generating, sustaining and/or propagating a macrophage is provided comprising the steps of isolating an MDSC; contacting the MDSC with an amount of a macrophage inducing agent such as lipopolysaccharide (LPS) effective to induce MDSC differentiation into a macrophage; and culturing the macrophage under conditions suitable for sustaining and/or propagating the macrophage.

In an additional aspect of the invention, a method of generating, sustaining and/or propagating a hepatocyte is provided comprising the steps of isolating an MDSC; contacting the MDSC with an amount of a hepatocyte inducing agent such as hepatocyte growth factor (HGF) effective to induce MDSC differentiation into a hepatocyte; and culturing the hepatocyte under conditions suitable for sustaining and/or propagating the hepatocyte.

Preferably, an MDSC of the invention is isolated from a mammalian source. Also preferred are human and adult human sources for the MDSC according to the invention.

The use of isolated MDSC for the treatment of various diseases and disorders is further contemplated by the invention. A disorder amenable to cell-based treatment includes, but is not limited to, Alzheimer's disease, Parkinson's disease, senile dementia, multiple sclerosis, age-related central nervous system (CNS) conditions, including changes manifested, e.g., as current time, date, location, or identity confusion, and/or recent memory loss, Acquired Immune Deficiency Syndrome (AIDS)-associated dementia, brain damage due to a blood clot, interruption of blood supply, formation or presence of a cyst, an autoimmune disorder, bacterial infection, e.g., of the brain, which may include an abscess, viral infection, e.g., of the brain, brain tumor, seizure disorders, neural trauma, surgical incision, diabetic ulcer, hemophiliac ulcer, varicose ulcer, solid angiogenic tumor, leukemia, hemangioma, acoustic neuroma, neurofibroma, trachoma, pyogenic granuloma, rheumatoid arthritis, psoriasis, diabetic retinopathy, retinopathy of premature macular degeneration, corneal graft rejection, neovascular glaucoma, retrolental fibroplasia, rubeosis, Osler-Webber Syndrome, myocardial angiogenesis blindness, plaque neovascularization, telangiectasia, hemophiliac joint, angiofibroma, wound granulation, epithelial cell neoplasia, Crohn's disease, chemical-, heat-, infection- or autoimmune-induced intestinal tract damage, chemical-, heat-, infection- or autoimmune-induced skin damage, systemic lupus erythematosus, AIDS, reactive arthritis, Lyme disease, insulin-dependent diabetes, an organ-specific autoimmune disorder, rheumatoid arthritis, inflammatory bowel disease, Hashimoto's thyroiditis, Grave's disease, contact dermatitis, psoriasis, graft rejection, graft-versus-host disease, sarcoidosis, a gastrointestinal allergy, eosinophilia, conjunctivitis, glomerular nephritis, a helminthic infection, lepromatous leprosy, diabetes, Gaucher's disease, Niemann-Pick disease, a parasitic infection, cancer, a disorder of the immune system, chemical (including drugs and alcohol)-, physical-, infection-, or autoimmune-induced hepatotoxicity, liver cancer, liver damage induced by metastatic cancer, and a liver blood clot.

According to the invention, the MDSC is preferably isolated from the organism to receive treatment (i.e., is an autologous MDSC). Preferably, the MDSC used to treat a disorder is derived from a mammalian, human, or adult human source.

The invention is further useful in treating a variety of diseases according to the methods described herein. One aspect of the invention provides a method for treating a neuronal disorder amenable to cell-based treatment comprising administering a pharmaceutically effective amount of an neuronal cell obtained by the methods described herein. A neuronal cell disorder amenable to cell-based treatment includes, but is not limited to, Alzheimer's disease, Parkinson's disease, senile dementia, multiple sclerosis, age-related CNS conditions, including changes manifested, e.g., as current time, date, location, or identity confusion, and/or recent memory loss, AIDS-associated dementia, brain damage due to a blood clot, an interruption of blood supply, formation or presence of a cyst, an autoimmune disorder, a bacterial infection including an abscess, a viral infection, e.g., of the brain, a brain tumor, a seizure disorder, and a neural trauma. It is further contemplated that a neuronal cell derived from an MDSC according to the invention may be used to ameliorate a symptom associated with an disorder amenable to cell-based treatment, as mentioned above, comprising administering a pharmaceutically effective amount of a neuronal cell obtained by the methods described herein. Symptoms associated with such disorders are well known in the art.

Another aspect of the invention is drawn to a method of treating an endothelial cell disorder amenable to cell-based treatment comprising administering a pharmaceutically effective amount of an endothelial cell obtained by the methods described herein. An endothelial cell disorder amenable to cell-based treatment includes, but is not limited to, a surgical incision, a diabetic ulcer, a hemophiliac ulcer, a varicose ulcer, a solid angiogenic tumor, a leukemia, a hemangioma, an acoustic neuroma, a neurofibroma, a trachoma, a pyogenic granuloma, rheumatoid arthritis, psoriasis, diabetic retinopathy, retinopathy of premature macular degeneration, a corneal graft rejection, a neovascular glaucoma, a retrolental fibroplasia, rubeosis, Osler-Webber Syndrome, myocardial angiogenesis blindness, plaque neovascularization, telangiectasia, a hemophiliac joint, an angiofibroma, and wound granulation. It is further contemplated that an endothelial cell derived from an MDSC according to the invention may be used to ameliorate a symptom associated with a disorder amenable to cell-based treatment, as mentioned above, comprising administering a pharmaceutically effective amount of an endothelial cell obtained by the methods described herein. Symptoms associated with such disorders are well known in the art.

Yet another aspect of the invention provides a method of treating an epithelial cell disorder amenable to cell-based treatment comprising administering a pharmaceutically effective amount of an epithelial cell obtained by the methods described herein. An epithelial cell disorder amenable to cell-based treatment includes, but is not limited to, an epithelial cell neoplasia, Crohn's disease, chemical-, heat-, infection- or autoimmune-induced intestinal tract damage, or chemical-, heat-infection and autoimmune-induced skin damage. It is further contemplated that an epithelial cell derived from an MDSC according to the invention may be used to ameliorate a symptom associated with a disorder amenable to cell-based treatment comprising administering a pharmaceutically effective amount of an epithelial cell obtained by the methods described herein. Symptoms associated with such disorders are well known in the art.

The invention further comprehends a method of treating a T-lymphocyte disorder amenable to cell-based treatment comprising administering a pharmaceutically effective amount of a T-lymphocyte obtained by the methods described herein. A T-lymphocyte disorder amenable to cell-based treatment includes, but is not limited to, leukemia, systemic lupus erythematosus, AIDS, Crohn's disease, reactive arthritis, Lyme disease, insulin-dependent diabetes, an organ-specific autoimmune disorder, rheumatoid arthritis, inflammatory bowel disease, Hashimoto's thyroiditis, Grave's disease, contact dermatitis, psoriasis, graft rejection, graft-versus-host disease, sarcoidosis, a gastrointestinal allergy, eosinophilia, conjunctivitis, glomerular nephritis, a helminthic infection, a viral infection, a bacterial infection and lepromatous leprosy. It is further contemplated that a T lymphocyte derived from an MDSC according to the invention may be used to ameliorate a symptom associated with a disorder amenable to cell-based treatment comprising administering a pharmaceutically effective amount of a T-lymphocyte obtained by the methods described herein. Symptoms associated with such disorders are well known in the art.

In yet another aspect of the invention, a method of treating a macrophage cell disorder amenable to cell-based treatment comprising administering a pharmaceutically effective amount of a macrophage obtained by the methods described herein. A macrophage cell disorder amenable to cell-based treatment includes, but is not limited to, diabetes, Gaucher's disease, Niemann-Pick disease, a bacterial infection, a parasitic infection, cancer, leukemia and a disorder of the immune system is provided. It is further contemplated that a macrophage derived from an MDSC according to the invention may be used to ameliorate a symptom associated with a disorder amenable to cell-based treatment comprising administering a pharmaceutically effective amount of a macrophage obtained by the methods described herein. Symptoms associated with such disorders are well known in the art.

In an additional aspect, the invention provides a method of treating a hepatocyte disorder amenable to cell-based treatment comprising administering a pharmaceutically effective amount of a hepatocyte obtained by the methods described herein. A hepatocyte disorder amenable to cell-based treatment includes, but is not limited to, chemical (including drugs and alcohol)-, physical-, infection-, or autoimmune-induced hepatotoxicity, liver cancer, liver damage induced by metastatic cancer, systemic lupus erythematosus, AIDS, Niemami-Pick disease, cancer, and a liver blood clot. It is further contemplated that a hepatocyte derived from an MDSC according to the invention may be used to ameliorate a symptom associated with a disorder amenable to cell-based treatment comprising administering a pharmaceutically effective amount of a hepatocyte obtained by the methods described herein. Symptoms associated with such disorders are well known in the art.

It is further contemplated that administration of one or more cell types according to the invention (e.g., MDSC and both non-terminally and terminally differentiated cells thereof) may be used to treat a disease or disorder or to ameliorate a symptom associated with such a disease or disorder.

Pharmaceutical compositions are also contemplated. Preferably, a pharmaceutical composition of the invention comprises a MDSC and a pharmaceutically acceptable diluent, carrier or medium. The invention further contemplates a kit comprising a pharmaceutical composition according to the invention.

Other features and advantages of the invention will be better understood upon review of the brief description of the drawing and the detailed description, which follow.

### BRIEF DESCRIPTION OF THE DRAWING

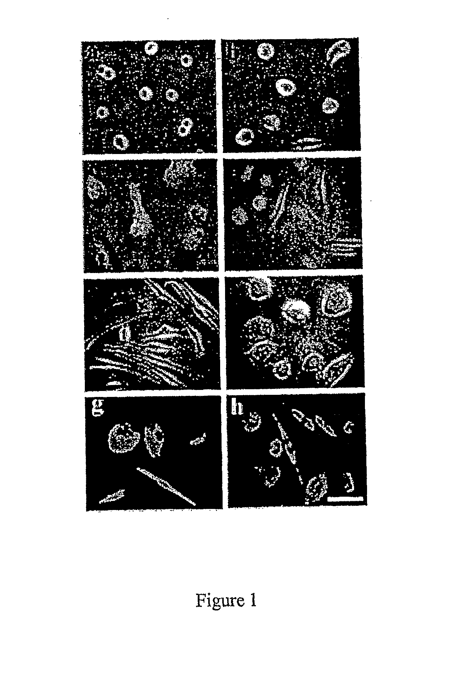

**Figure 1****.** Macrophage differentiation of peripheral blood monocytes and MDSC growth; a) freshly isolated monocytes, b) untreated 5-day-old monocyte culture, c) 5-day PMA-treated monocyte culture, d) 5-day M-CSF-treated monocyte culture, e) 14-day M-CSF-treated monocyte culture; the arrow points to a dividing cell, f) 14-day M-CSF-treated monocyte culture incubated for 1 day with LPS, g) MAC-1 immunostaining of 5 day M-CSF-treated monocyte culture, and h) fluorescence of phagocytized beads in 5-day M-CSF-treated monocyte culture. For Fig. 1a-f, cells were visualized by phase-contrast microscopy merged with fluorescence images of lipids stained with Nile red (red) and nuclei stained with 4',6-diamidino-2-phenylindole (DAPI, blue). Scale bar, 40 µm.

**Figure. 2****.** Replication of MDSCs. MDSCs in untreated (x-x) and M-CSF-treated (●-●) monocyte cultures, and s-MΦ (S-macrophage or standard macrophage) in untreated (▲-▲), and M-CSF-treated (■-■) monocyte cultures. The results are the mean ± s. d. of cell counts from 4 different individuals.

**Figure. 3****.** LPS-induced macrophage differentiation of MDSCs.
Fluorescence intensity (mean ± s.d. of 4 experiments) is based on 30-50 cells/determination/individual.

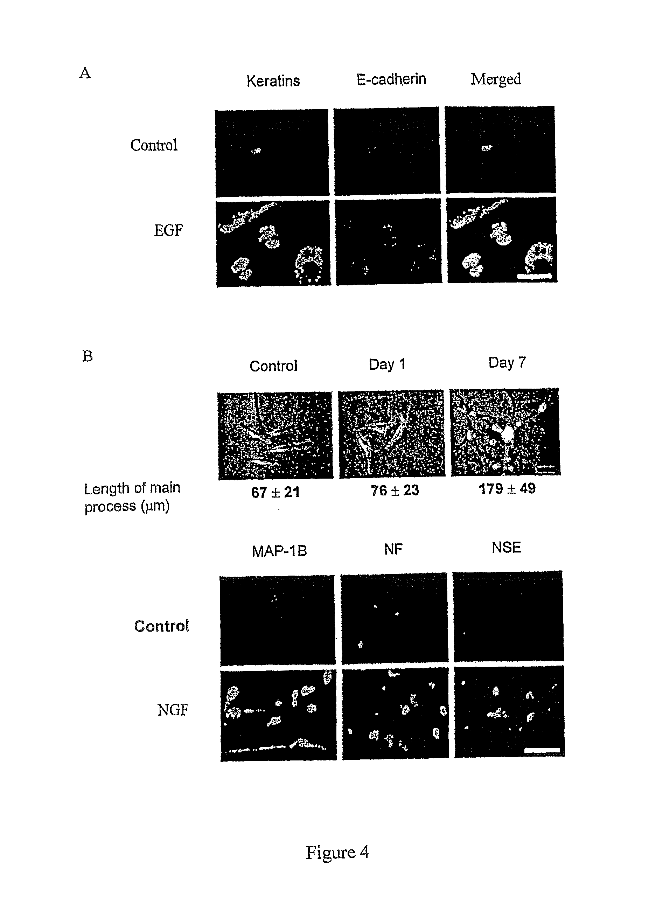

**Figure. 4****.** Epithelial and neuronal cell differentiation of MDSCs. A, EGF-induced epithelial cell differentiation was assessed by double immunostaining for keratins (green) and E-cadherin (red). Each field contains 4-5 cells. The control panel was selected to include a positive cell. B, bNGF-induced neuronal cell differentiation was assessed by length of the main processes (mean ± s.d.) of 50 randomly selected cells using Slidebook software (upper panel) and by immunostaining for neuron-specific antigens (lower panel). Each immunostained field contains 10-15 cells with the control panel selected to contain positive cells. Scale bar, 50 µm. MAP-1B, microtubule-associated protein-1B; NF, neurofilament; NSE, neuron-specific enolase.

**Figure. 5****.** Relative cell number in MDSC cultures treated with or without differentiation inducers. The results are the mean ± s.d. of 5 randomly selected microscopic fields, each from 4 different experiments for each treatment.

### DETAILED DESCRIPTION OF THE INVENTION

The invention provides pluripotent adult stem cells derivable from peripheral blood sources, as well as methods for culturing, propagating and/or differentiating such cells. The invention also provides methods of using such cells to treat any of a variety of disorders or diseases, or to ameliorate at least one symptom of one or more such disorders or diseases. The pluripotent adult stem cells of the invention are a subset of monocytes and are preferably obtained from humans, domesticated livestock, or pets. The cells of this subset are herein identified as monocyte-derived stem cells (MDSCs). The examples provided herein demonstrate that an MDSC can be induced to differentiate into a variety of non-terminally or terminally differentiated cells, including macrophage, T-lymphocyte, epithelial cell, endothelial cell, neuronal cell, or hepatocyte (i.e., to acquire a phenotype characteristic of such a cell).

One advantage of the invention is the capability to administer autologous MDSCs, and/or cells differentiated therefrom, to patients in need of such cells. The use of autologous MDSCs or their progeny reduces the risk of immune rejection and the transmission of disease. Further, the ability to propagate autologous MDSCs, thereby producing useful quantities of those cells, is expected to expand the number and variety of disorders and diseases amenable to therapies (and the number and variety of symptoms thereof amenable to amelioration) based on MDSC administration. Thus, methods of the invention show promise in being more effective and versatile than current procedures, which do not include such an expansion of cells. The dosage and manner of administration are readily determinable by one of skill in the art using nothing more than routine optimization, with such efforts being guided by the type of cells being administered (MDSCs and/or derivatives thereof). Thus, the ability to store, propagate and differentiate the MDSCs make them invaluable for autologous administration.

Advantages of the invention include the use of peripheral blood as a convenient source for MDSCs, including autologous MDSCs, which can be safely and economically obtained. Further, it is generally appreciated in the art that peripheral blood is readily renewable and can provide a continuing source of autologous, or heterologous, pluripotent stem cells. A further advantage of the invention is that the blood source for MDSC preparation may be an adult source. As such, the controversial sampling of embryonic stem cells is avoided. Moreover, the adult blood source may be the very patient requiring administration of MDSCs or cells derived therefrom. To better understand the invention, the following definitions are provided.

"Adult" or "adult human" means a mature organism or a mature cell such as a mature human or a mature human cell, regardless of age, as would be understood in the art.

The term "stem cell" refers to any cell that has the ability to differentiate into a variety of cell types, including terminally differentiated cell types. Such cells are, therefore, properly regarded as progenitor cells. Stem cells can be pluripotent, i.e., capable of differentiating into a plurality of cell types.

As defined herein, the term "isolated" refers to cells that have been removed from their natural environment, typically the body of a mammal. Preferably, isolated cells are separated from other cell types such that the sample is homogeneous or substantially homogeneous. As a specific example, a blood cell monocyte is isolated if it is contained in a sample of blood that has been removed from an organism.

"Monocyte-derived stem cell" or "MDSC" means stem cell derived from the monocyte fraction of the blood. "Peripheral blood monocyte" or "PBM" means a monocyte cell typically found in the peripheral blood of a vertebrate such as a mammal. These definitions comport with the ordinary and accustomed meanings of these cell-based terms in the art.

"Surface antigen" means a compound, typically proteinaceous, that is capable of binding to an antibody and is typically localized to a cell surface, such as by association with a cell membrane. A cell "marker," such as an "adipocyte marker," is a detectable element sufficiently associated with a cell, such as an adipocyte, as to be characteristic of that cell or cell type. One class of useful markers is cell-surface markers, which can be detected with minimal disruption of cellular activity.

Cell-based "activity" refers to a function(s) of a given cell or cell type. One category of useful activities is the activities useful in distinguishing a given cell or cell type from other cells or cell types. For example, an activity of a macrophage is phagocytosis, which is a distinguishing characteristic of macrophages.

"Cytokine" is given its ordinary and accustomed meaning of a regulatory protein released by a cell usually of the immune system that acts as an intercellular mediator in the generation of a cellular response such as an immune response. Examples of cytokines are the interleukins and lymphokines.

"Dispersion" means dissolution, i.e., to loosen or dissociate. As used herein, dispersion is not limited to dissolving or forming a solution thereof. In the context of the invention, the dissociation of cells, or a cell and a solid surface, typically a solid surface available to the cell during cell culture or propagation.

"Vertebrate" is given its ordinary and accustomed meaning of any organism properly characterized as having a bony or cartilaginous backbone made of vertebra. Similarly, the term "mammalian," as defined herein, refers to any vertebrate animal, including monotremes, both marsupial and placental, that suckle their young and either give birth to living young (eutharian or placental mammals) or are egg-laying (metatharian or nonplacental mammals). Examples of mammalian species include primates (e.g., humans, monkeys, chimpanzees, baboons), rodents (e.g., rats, mice, guinea pigs, hamsters, rabbits), ruminants (e.g., cows, horses, sheep), canines (e.g., dogs, wolves) and felines (e.g., lions, tigers, cats).

By "suitable conditions" for growth, propagation or culture, it is meant that the temperature, humidity, oxygen tension, medium component concentrations, time of incubation and relative concentrations of cells and growth factors are at values compatible with the generation of progeny or sustaining cell viability. Each of the variables involved in cell growth or culture is well known in the art and, generally, a range of suitable values can be obtained using routine experimentation to optimize each result-effective variable.

The term "growth" is given its ordinary and accustomed meaning of the expansion of a cell population and/or cell size. Thus, the term "growth factor" as defined herein refers to a compound that is capable of inducing, or modifying the rate of, cell growth.

A cell "culture" is one or more cells within a defined boundary such that the cell(s) are allotted space and growth conditions typically compatible with cell growth or sustaining its viability. Likewise, the term "culture," used as a verb, refers to the process of providing said space and growth conditions suitable for growth of a cell or sustaining its viability.

The term "propagate" or "propagation" refers to the process of cell growth. A "mitogenic compound" is a compound capable of affecting the rate of cell division for at least one cell type under at least one set of conditions suitable for growth or culture.

The phrase "disorder amenable to cell-based treatment" refers to a disorder that can be treated in whole or in part by administration of cells, whether autologous or heterologous to the recipient. The definition further embraces those disorders characterized by an effective cell deficiency (e.g., deficiency in number of cells or deficiency in number of healthy cells) as well as those disorders resulting from an abnormal extracellular signal wherein the administered cells can modulate/affect the level of that signal. As such, the definition embraces the physical re-supplying of cells and/or taking advantage of the physiology of the administered cells to restore an extracellular signal to levels characteristic of, or approaching that of, healthy individuals.

The term "differentiation" is given its ordinary and accustomed meaning of the process by which a cell or cells change to a different and phenotypically distinct cell type. A "differentiation inducer" is a compound that is a direct, or indirect, causative agent of the process of cell differentiation. Using this definition, a "differentiation inducer" is not be essential to differentiation.

An "inducing agent" or inducer is a differentiation inducer, i.e., a substance capable of directing, facilitating or promoting at least one type of cellular differentiation.

An "age-related CNS change" means a central nervous system alteration or change as manifested by confusion regarding the current time, the current date, the current location, self-identity, recent memory loss, or one or more other common facts that are well known and provide a basis for assessing the mental state of humans.

An "effective" or "pharmaceutically effective" amount is that amount that is associated with a desired effect, for example a pharmaceutical effect. Typically in the context of the invention, it is that amount or number of MDSCs (and/or differentiated MDSC derivatives) which, when administered using conventional techniques, will result in a beneficial effect on a disorder or disease, or a symptom associated therewith, without unacceptably deleterious effects on the health or well being of the animal or human patient. By way of example, an effective amount is that amount of M-CSF that causes PBM propagation, and particularly MDSC propagation, preferably increasing the relative contribution of MDSCs to such cultures. A pharmaceutically effective amount, by way of example, is that amount of neuronal cells derived from MDSCs that will ameliorate a symptom of Alzheimer's disease.

"Viability" is given its ordinary and accustomed meaning of a state characterized by the capacity for living, developing or germinating. In context, "viability" refers to the state of a cell. Measures of viability include, but are not limited to, a determination of the absolute, or relative, number(s) of cells, or an assessment of the absolute or relative health of one or more cells, using any one or more characteristic or property of a cell recognized in the art as informative on the health of a cell.

In view of the preceding definitions, one of ordinary skill in the art will understand that the invention provides methods for preparing an isolated MDSC that comprise the steps of (a) isolating a peripheral-blood monocyte (PBM), (b) contacting the PBM with an effective amount of a mitogenic compound selected from the group consisting of macrophage colony-stimulating factor (M-CSF), interleukin-6 (IL-6) and leukemia inhibitory factor (LIF), and (c) culturing the PBM under conditions suitable for propagation of said cell, thereby obtaining a preparation of an isolated MDSC.

An isolated PBM is incubated with an effective amount of M-CSF (25-200 ng/ml), IL-6 (10-50 ng/ml) or LIF (100-2000 units/ml) according to one aspect of the invention. Preferably, 50 ng/ml M-CSF, 20 ng/ml IL-6 or 1000 units/ml LIF is used to treat preparations of cultured human PBM.

The M-CSF, IL-6 or LIF used in the invention may be from any suitable source, such as a natural or synthetic source, and may be used in a purified or unpurified state. Further, it is contemplated that the M-CSF, IL-6 or LIF may be a holoprotein or may be active subunits or fragments that exhibit a mitogenic effect on PBMs. Similarly, the M-CSF, IL-6 or LIF maybe used alone or in combination (e.g., with other mitogens), with suitable buffers and the like. The use of conventional assays may be used to determine the quantity and dosage of M-CSF, IL-6 or LIF associated with a sufficient mitogenic effect.

According to methods of the invention, PBMs are incubated with one or more growth factors (i.e., mitogenic compounds) under suitable growth conditions to propagate MDSCs. Likewise, the MDSC of the invention is incubated with one or more of various differentiation inducers (i.e., inducers or inducing agents), and optionally one or more growth factors, under suitable conditions to allow for differentiation, and optionally propagation, of a variety of cell types. As one of skill would recognize, there are known compounds that function as both growth factors and differentiation inducers. Growth factors of the invention include, but are not limited to, macrophage-colony stimulating growth factor (M-CSF), interleukin-6 (IL-6) and leukemia inhibitory factor (LIF). Examples of compounds functioning as growth factors and/or differentiation inducers include, but are not limited to, lipopolysaccharide (LPS), phorbol 12-myristate 13-acetate (PMA), stem cell growth factor, human recombinant interleukin-2 (IL-2), IL-3, epidermal growth factor (EGF), b-nerve growth factor (NGF), recombinant human vascular endothelial growth factors isoform (VEGF), and hepatocyte growth factor (HGF). Useful doses for inducing MDSC differentiation by growth and/or differentiation factors are: 0.5-1.0 µg/ml (preferably 1.0 µg/ml) for LPS, 1-160 nM (preferably 3 nM) for PMA, 500-2400 units/ml (preferably 1200 units/ml) for IL-2 , 50-1,600 ng/ml (preferably 200 ng/ml) for bNGF, 12.5-100 ng/ml (preferably 50 ng/ml) for VEGF, 10-200 ng/ml (preferably 100 ng/ml) for EGF, and 25-200 ng/ml (preferably 50 ng/ml) for HGF.

Cell surface antigens and cell markers may be identified using any technique known in the art, including immunostaining. Surface antigens and markers which, alone or in combination, are characteristic of cells according to the invention include MAC-1, CD14, CD34, CD40 and C45, whereas CD1a and CD83 are characteristically not associated with cells according to the invention. By way of example, cell surface antigens or markers have been identified using cells on glass slides, the cells having been immunostained by washing with phosphate-buffered saline (PBS) and fixed with 4% formaldehyde in PBS for 20 minutes at 20°C. For intracellular proteins, the cells were permeabilized with 0.5% Triton X-100 for 5 minutes at 20°C and incubated for one hour with the primary antibodies. The primary antibodies were diluted with PBS containing 1% BSA to block non-specific reactivity. The cells were then washed 3 times with PBS containing 1% BSA and incubated for 45 minutes with FITC-, TRITC-, or Cy5-conjugated cross-adsorbed donkey secondary antibodies (Jackson ImmunoResearch, West Grove, PA). Both of these reactions were performed at saturating concentrations and at 4°C. The slides were then washed and mounted with phosphate-buffered gelvatol.

Fluorescence imaging may be used to monitor or detect cells and is performed using techniques known in the art. For example, automated excitation and emission filter wheels, a quad-pass cube, and SlideBook software may be used for fluorescence imaging. Quantitative fluorescence ratio imaging can be performed using glyceraldehyde 3-phosphate dehydrogenase immunofluorescence (sheep polyclonal antibody, Cortex Biochem., San Leonardo, CA) as an internal standard. The fluorescence intensity level detected after reacting a sample with an isotype-matched IgG antibody provides a background fluorescence level, which is primarily attributable to non-specific binding. This fluorescence intensity was arbitrarily assigned an intensity level of one.

Among the antibodies contemplated for use in the invention are mouse monoclonal antibodies to IL-1β, IL-6, IL-10, CD14, CD34, CD40, CD45, HLA-DR, HLA-DQ, CD1a, CD83, von Willebrand's factor (vWF), keratins (Pan Ab-1), cytokeratin 7, α-fetoprotein (AFP), microtubule-associated protein-1B (MAP-1B), neurofilament Ab-1 (NF), IL-12p70, tumor necrosis factor-α (TNF-α), TNF-α receptor I (TNF-RI) and TNF-RII. Further, mouse IgG₁, IgG_{2A}, IgG_{2B}, and goat IgG antibody to CD3, CD4, CD8 and human albumin; rat monoclonal antibody to E-cadherin; rabbit polyclonal antibodies to neuron-specific enolase (NSE), peroxisome proliferator-activated receptor (PPAR)y2, IL-6, leptin and VEGF-R3 (FLT-4), and mouse monoclonal antibody to VEGF-R2 (FLK-1) are also contemplated for use in the invention.

Upon incubation with the appropriate differentiation inducer, an MDSC of the invention has the ability to differentiate into a variety of cell types. For example, according to methods of the invention, following contact by an effective amount of bNGF, an MDSC differentiates into a neuronal cell when under suitable growth conditions. In one embodiment, 200 ng/ml bNGF was used to treat MDSC cultures. It is contemplated by the invention that inducers of neuronal cell differentiation known in the art may be used under growth conditions and inducer concentrations that allow for optimal differentiation. These may include, but are not limited to, NGF, brain-derived neurotrophic factor, neurotrophin-3, basic fibroblast growth factor, pigment epithelium-derived factor, or retinoic acid.

According to other methods of the invention, endothelial cells are prepared by contacting MDSCs with VEGF under suitable growth conditions. In one embodiment, 50 ng/ml of VEGF was used to treat cultures of MDSC for 5-7 days. However, it is contemplated by the invention that other known inducers of endothelial cell differentiation may be substituted for VEGF. These may include, but are not limited to, insulin growth factor and basic fibroblast growth factor.

Analogously, the invention provides methods to prepare epithelial cells by contacting MDSCs with EGF under suitable culture conditions. By way of example, 100 ng/ml EGF was incubated with an MDSC sample for 4 days. However, it is contemplated by the invention that other known inducers of epithelial cell differentiation may be substituted for EGF. These include, but are not limited to, bone morphogenesis protein-4, elevated calcium concentrations, retinoic acid, sodium butyrate, vitamin C, hexamethylene bis acetate, phorbol 12-myristate 13-acetate (PMA), teleocidin, interferon gamma, staurosporin, or activin.

According to still other methods of the invention, a macrophage and/or a T-lymphocyte is prepared by contacting an MDSC with an appropriate inducer, such as LPS, for macrophage development and IL-2 for T-lymphocyte development. For example, 1 µg/ml LPS and 1200 units/ml IL-2 are incubated with MDSCs to achieve macrophage and T-lymphocyte cell differentiation, respectively. It is contemplated by the invention that other known inducers of macrophage and T-lymphocyte cell differentiation may be substituted for LPS and IL-2. These may include, but are not limited to, IL-4, IL-12, IL-18, CD3 antibody, PMA, teleocidin, or interferon gamma.

In a similar way, the invention provides methods to prepare hepatocytes by contacting MDSCs with human recombinant hepatocyte growth factor (HGF) under suitable culture conditions. By way of example, 50 ng/ml of HGF is incubated with an MDSC sample for 5-7 days. However, it is contemplated by the invention that other known inducers of hepatocyte differentiation may be substituted for HGF. These include, but are not limited to, retinoic acid, oncostatin M, phenobarbital, dimethyl sulfoxide, dexamethasone, or dexamethasone and dibutyryl cyclic AMP.

The currently described MDSC and/or cell derived therefrom is, among other uses, employed to replenish a cell population that has been reduced or eradicated by a disease or disorder (e.g., cancer), by a treatment for such a disease or disorder (e.g., a cancer therapy), or to replace damaged or missing cells or tissue(s). By way of example, neuronal tissue damaged during the progression of Parkinson's disease, endothelial cells damaged by surgical incisions, macrophage cells affected by Gaucher's disease, epithelial cells damaged from skin burns, T-lymphocytes affected by Lyme disease or hepatocytes damaged as a result of cirrhosis, are replenished by cells according to the invention. In addition, individuals with congenital diseases can be engrafted with autologous MDSCs or their progeny, after repairing the genetic alteration or further modifying the genome (e.g., introduction, deletion or modification of an expression control sequence, introduction of a modification in the genome that functions as a second-site reversion, and the like) by recombinant technology. Moreover, the ability to propagate autologous MDSCs *in vitro* before administration of such cells should yield a sufficient number of stem cells for this procedure, which is expected to be more effective and versatile than the current transplantation procedures that do not include such an expansion.

The invention is illustrated by the following examples, which are not intended to be limiting in any way. The examples show that an MDSC, derived from a peripheral blood source, can be induced to differentiate into, or acquire a characteristic phenotype of, a macrophage, lymphocyte, epithelial cell, endothelial cell, neuronal cell or hepatocyte phenotype. Briefly, Example 1 describes the isolation and storage at -70°C of adult human monocytes from peripheral blood and the culturing of MDSCs. Examples 2-7 describe the verification of differences between s-MΦ and MDSCs (Example 2), and the differentiation of MDSCs to macrophages and T-lymphocytes (Example 3), epithelial cells (Example 4), neuronal cells (Example 5), endothelial cells (Example 6), and hepatocytes (Example 7). Example 8 describes a clonal analysis to determine whether single monocytes generate colonies of MDSCs whose progeny is capable of, at least, T-lymphocyte, epithelial, neuronal, endothelial and hepatocyte differentiation.

### Example 1

### Isolation and culturing of adult human MDSC from peripheral blood

Peripheral blood monocyte (PBM) preparations from about 50 ml buffy coats samples (each from 500 ml peripheral blood) of healthy individuals (LifeSource Blood Services, Glenview, IL) were obtained by a selective attachment method as previously described (Hoklland, M. et al., Cell Biology, a laboratory handbook, Celis J. E. ed., Academic Press, 1: 179-181(1994)). Buffy coat cell samples of 20-25 ml, which were diluted earlier with an equal volume of RPMI 1640 medium (Life Technologies, Inc.), were carefully layered over 20 ml Ficoll-Hypaque (γ=1.077) in 50 ml centrifuge tubes and then centrifuged using a Beckman CPKR centrifuge and a GH-3.7 horizontal rotor at 3,500 rpm (2700g) for 25 minutes at 4°C. After carefully harvesting the mononuclear cells at the interface, cells were washed 2-3 times with RPMI 1640 medium by centrifugation using a Beckman CPKR centrifuge and a GH-3.7 horizontal rotor at 1,000 rpm (250g) for 10 minutes. The cells were then used for culture and/or stored in liquid nitrogen in a 90% bovine calf serum and 10% dimethyl sulfoxide solution. The cells, including those obtained from storage in liquid nitrogen, were incubated at 2-3 x 10⁷ cells/15 cm dish. After 8-12 hours incubation at 37°C (8% CO₂), the floating cells were removed and the dishes were rinsed 5 times with RPMI 1640 medium. The attached cells were then detached from the surface of the dishes by forceful pipetting with 5-10 ml of RPMI 1640 medium supplemented with 10% bovine calf serum.

The percentage of PBM was verified by immunostaining with an R-phycoerythin-conjugated mouse anti-human CD14 monoclonal antibody using a Becton Dickinson FACS can. The fraction of CD14 cells in these cell preparations, which were usually used in the experiments, was 90-95%. In a number of experiments, the CD14-immunostained cells were further isolated to a purity of 99.97% by using a droplet cell-sorting method by means of a 5 detector Becton Dickinson FACStarPlus Cell Sorter. The isolated PBMs were inoculated at 1x10⁵ cells/ml in 8-well LabTek chamber slides (Nunc, Inc., Naperville, IL) at 0.4 ml/well in a 37°C humidified atmosphere containing 8% CO₂. Every five to seven days, one-half of the culture medium was replaced with fresh growth medium. This medium consisted of RPMI-1640 supplemented with 10% heat-inactivated bovine calf serum (Harlan, Indianapolis, IN), 100 units/ml penicillin, 100 µg/ml streptomycin, and 2 mM L-glutamine (Life Technologies).

Five preparations of cultured human peripheral blood monocytes, each from a different individual, were treated with 50 ng/ml M-CSF (Zhoa et al., Proc. Natl. Acad. Sci., 100:2426-2431 (2003); incorporated herein by reference in its entirety). After five days of incubation, the cultures contained two major morphologically distinct subsets of cells. The less abundant of the two subsets, containing about 25-35% of the total cells, was composed of elongated cells that morphologically resembled fibroblasts and were termed monocyte-derived stem cells (MDSCs). The other subset, containing about 65-75% of the total, was composed of standard macrophages, which were termed s-macrophages or standard macrophages (s-MΦ) (Fig.1). Liquid nitrogen-stored PBMs from two of the five individuals yielded similar results. Two other PBM preparations, including one obtained from liquid nitrogen, each from a different individual, were incubated with any one of 50 ng/ml M-CSF, 1000 units/ml LIF, 20 ng/ml IL-6, or a combination of M-CSF with either LIF or IL-6 (Table 1). After five days, the cultures treated with M-CSF or LIF yielded about 30% MDSCs, while the cells treated with IL-6 contained about 20% of these cells. Treatment with both M-CSF and LIF displayed an approximately additive effect, namely, the cultures were composed of about 50% MDSCs. Incubation with both M-CSF and IL-6 failed to yield such an effect (Table 1). Significantly, control cultures had only about 5% of these cells (Table 1). Both the MDSCs and s-MΦ displayed an ability to attach and spread on culture matrices, engulf fluorescent beads and express MAC-1 (Fig. 1), each of which are characteristic markers of macrophage (Laouar et al., Cell Biology: A Laboratory Hand book, J. E. Cells Ed., Academic Press, vol. 1, 233 (1997); Schlossman, S. et al. Eds., Leukocyte Typing V: White Cell Differentiation Antigens, Oxford Univ. Press, New York (1995)).

Macrophages are known to function as antigen-presenting cells and as such they produce cytokines and display characteristic cell-surface molecules (Gordon et al., Curr. Opin. Immunol., 7: 24-33 (1995); Martinez-Pomares et al., Immunobiology, 195: 407-416 (1996); Grage-Griebenow et al., J. Leukoc. Biol. 69:11-20 (2001)). Immunostaining for these proteins indicated that both cell types share some of the characteristics of antigen-presenting cells. However, the MDSCs differed from s-MΦ in that they exhibited reduced levels of IL-10, TNF-α, TNFRII, CD1a, HLA-DR and HLA-DQ (Table 2). In Table 2, fluorescence intensities of cell-surface antigens, cytokines, leptin and PPARγ2 were determined after immunostaining, and lipid droplets were assessed after Nile red staining. Relative fluorescence intensity was examined by quantitative ratio imaging microscopy. Stimulation of lymphocyte proliferation was performed using a 10:1 macrophage to lymphocyte ratio and cytotoxicity was assessed using a 5:1 macrophage to target cell ratio, as previously described (Nakabo et al., J. Leukoc. Biol., 60:328-336 (1996), Zhou et al., Proc. Natl. Acad. Sci. USA, 93:2588-2592 (1996)). The MDSCs were found to be less cytotoxic to human leukemia cells and were more effective than s-MΦ cells in stimulating lymphocyte proliferation (Table 2). Another property that distinguished MDSCs from s-MΦ was their reduced ability to express leptin and PPARγ2 (Tontonoz et al., Cell, 93:241-252 (1998)) and their increased susceptibility to staining for lipid droplets (Fig. 1d, Table 2).

**Table 1**

| **Treatment** | **MDSC (%)** |
|---|---|
| **Control** | 5 ± 3 |
| M-CSF (50 ng/ml) | 35 ± 8 |
| LIF (1,000 units/ml) | 26 ± 7 |
| IL-6 (20 ng/ml) | 17 ± 4 |
| M-CSF + LIF | 49 ± 11 |
| M-CSF + IL-6 | 27 ± 9 |

**Table 2**

| | **MDSC** | **s-MΦ** |
|---|---|---|
| **Surface antigens** | **Relative fluorescence intensity** | |
| MAC-1 | 69 ± 11 | 67 ± 7 |
| HLA-DR | 18 ± 4 | 106 ± 41 |
| HLA-DQ | 17 ± 5 | 83 ± 28 |
| CD1a | 1 | 15 ± 3 |
| CD14 | 129 ± 27 | 155 ± 22 |
| CD34 | 72 ± 19 | 21 ± 7 |
| CD40 | 49 ± 23 | 37 ± 18 |
| CD45 | 132 ± 27 | 144 ± 36 |
| CD83 | 1 | 1 |

| **Cytokine production** | | |
|---|---|---|
| IL-1β | 81 ± 29 | 78 ± 17 |
| IL-6 | 44 ± 18 | 59 ± 17 |
| IL-10 | 11 ± 6 | 53 ± 10 |
| IL-12 p70 | 54 ± 32 | 53 ± 8 |
| TNFα, | 25 ± 11 | 66 ± 17 |
| TNF-RI | 28 ± 6 | 30 ± 18 |
| TNF-RII | 8 ± 5 | 52 ± 19 |

| **Adipocyte markers** | | |
|---|---|---|
| Lipids | 17 ± 10 | 147 ± 10 |
| Leptin production | 25 ± 6 | 82 ± 18 |
| PPARγ2 | 21 ± 5 | 105 ± 31 |

| **Functional indicators** | | |
|---|---|---|
| Phagocytosis | 184 ± 18 | 191 ± 20 |
| Lymphocyte stimulation (A₅₄₀)* | 0.74 ± 0.05 | 0.17 ± 0.02 |
| Cytotoxicity (%) | 11 ± 3 | 68 ± 6 |

| | | |
|---|---|---|
| *A₅₄₀: optical absorbance at 540 nm. | | |

Thus, the MDSC of the invention can be isolated from peripheral blood samples of adults and can be distinguished from a variety of other cell types, whether native to the source organism or not. Further, the results demonstrated that storage of the PBM preparations in liquid nitrogen does not compromise the ability of the PBMs to differentiate to MDSCs, indicating that long-term freezing of the PBM preparations for the generation of a cell bank is possible. It is contemplated that cryopreservation of the MDSCs themselves, as well as cells terminally differentiated therefrom, will allow re-population of cells depleted from treatment of various diseases (e.g., following anti-cancer chemotherapy or radiation treatment).

One of ordinary skill in the art will appreciate that cells exhibiting one or more of the characteristics disclosed in Table 2 can be isolated from different sources of peripheral blood using routine techniques well known in the art.

### Example 2

### Verification of s-MΦ and MDSCs as two distinct cell types

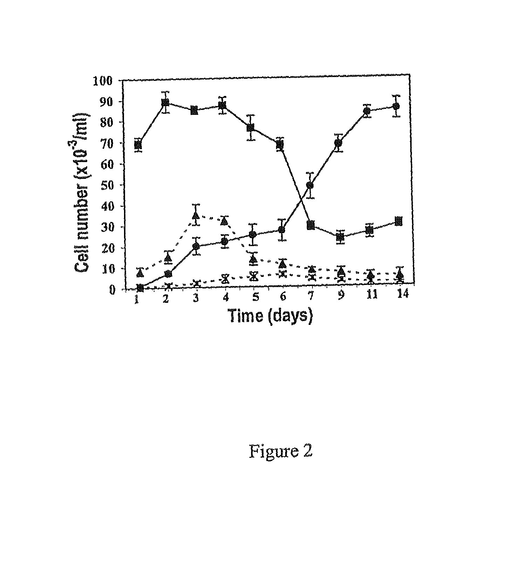

Unlike s-MΦ, MDSCs contained dividing cells (Fig. 1e) and displayed elevated levels of the hematopoietic stem cell marker CD34 (Randall et al., Stem Cells, 16:38-48 (1998))) (Table 1). In order to determine whether the MDSCs were simply replicating progenitors of s-MΦ, five preparations of cultured peripheral blood monocytes, each from a different human, were treated with 50 ng/ml M-CSF and the number of MDSCs and s-MΦ were determined over a period of 14 days by morphological examination. The results indicated that after 6 days, the number of MDSC increased while the number of s-MΦ decreased (Fig. 2). Based on the growth curve during this time, it was estimated that the MDSC population replicated about every three days. After day 10, the confluent cultures were composed of 80-90% MDSCs (Fig. 2). No such increase was observed in cultures untreated with M-CSF (Fig. 2). Replenishing the cultures with fresh M-CSF on days 5 or 12 had little impact on the appearance or number of MDSCs.

In this example, a feature of the MDSCs is resistance to dispersion by trypsin and/or EDTA, or dispase. Standard digestion with trypsin, trypsin-EDTA or dispase for up to 60 minutes failed to remove the MDSCs, which were tightly bound to the surface of the culture dish. Therefore, to obtain cell suspensions for subculture, the MDSCs were dispersed by forceful pipetting after incubation with 2% lidocaine in a PBS solution for 5-8 minutes, with the exception of the work described in Example 8.

Thus, the MDSCs of the invention are distinguishable from other cells (e.g., s-MΦ) found in peripheral blood. It will be appreciated by one of ordinary skill in the art that mitogenic compounds other than M-CSF, LIF or IL-6 may be used to propagate MDSCs. Further, a skilled artisan will recognize that various growth conditions may be used to the propagate stem cells. Moreover, it is within the skill in the art to optimize result-effective variables of culturing or propagation to optimize or maximize the propagation of MDSCs and its derivatives. Additionally, while the characteristics of MDSCs disclosed herein are sufficient to distinguish these cells from other cell types, it is expected that additional identifying characteristics of MDSCs will be found by those of skill in the art using routine procedures.

### Example 3

### Macrophage and T-lymphocyte cell differentiation

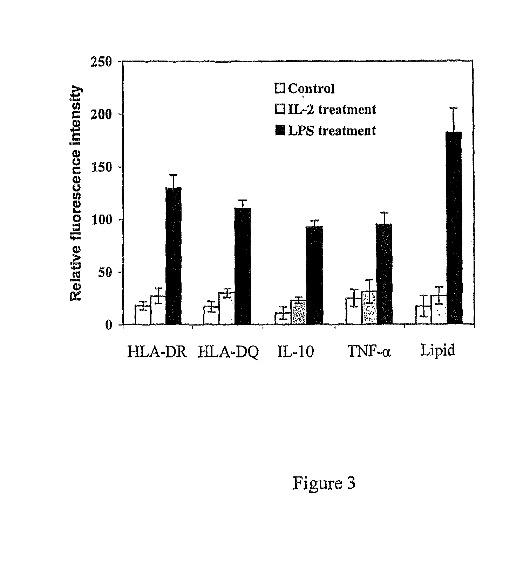

To confirm their progenitor nature (i.e., their pluripotency), preparations of 12-14-day-old, M-CSF-treated, monocyte cultures containing 80-90% MDSCs, from each of four different humans (MDSC cultures), were incubated with 1 µg/ml LPS, a macrophage activator (Vadiveloo et al., J. Leukoc. Biol., 66:579-582 (1999)). This treatment transformed the MDSCs into standard macrophages. This transformation was verified by characterization of morphology, lipid staining, increased HLA-DR, HLA-DQ, IL-10 and TNF-α immunostaining (Fig. 3), and cytotoxicity (Table 1).

To determine whether the MDSCs could also be induced to mature along another blood lineage, the ability of IL-2 to induce T-lymphocyte differentiation was tested. Treatment of four MDSC cultures with 1200 units/ml IL-2 for 4 days induced the cells to acquire a round morphology. This treatment also caused about 90% of the treated cells to express CD3, which is a defining characteristic of mature T-lymphocytes (Schlossman et al., Eds., Leukocyte Typing V: White Cell Differentiation Antigens (Oxford Univ. Press, New York 1995). Roughly 75% of the CD3-positive cells also displayed CD8, which characterizes cytotoxic/suppressor T lymphocytes (Ryffel et al., Proc. Natl. Acad. Sci. USA, 79:7336-7340 (1982); Lederman et al., Hum. Immunol., 60:533-561 (1999)). Control cultures contained 3-4% of cells that stained for CD3 and CD8. Less than 3% of control or IL-2-treated cells exhibited CD4, a helper T-lymphocyte marker (Schlossman et al., Eds., Leukocyte Typing V: White Cell Differentiation Antigens (Oxford Univ. Press, New York 1995). The IL-2-induced cells also acquired an increased ability to kill target cells, a functional marker for cytotoxic/suppressor T-lymphocytes. Using a 5:1 effector to target cell ratio, the IL-2-induced lymphocytes lysed 35 ± 7% of the target cells compared to 12 ± 3% by control cells.

Thus, MDSCs of the invention can be induced to differentiate into macrophages or various T-cell lymphocytes by exposure to effective quantities of LPS or IL-2, respectively. One of skill in the art will recognize that other known inducers of macrophage or T-cell differentiation may be substituted for the exemplified inducing compounds, LPS and IL-2. Moreover, skilled artisans will appreciate that suitable dosages of the inducing compounds can be determined using routine techniques well known in the art. It is further expected that known differentiation inducers of any of a wide variety of cell types will result in differentiation of MDSCs into such cell types, and the range of these differentiation inductions is illustrated by this example and the examples that follow.

### Example 4

### Epithelial cell differentiation

To determine whether MDSCs differentiate into lineages other than those of blood cells, the ability to differentiate into epithelial cells was initially tested. Four MDSC cultures prepared as described above were treated for 4 days with 100 ng/ml epithelial growth factor (EGF), a promoter of epithelial cell growth and differentiation (Carpenter et al., Curr. Opin. Cell Biol., 5:261-264 (1993)). This treatment induced about 70% of the MDSCs to display an epithelial cell morphology. This treatment also caused 71 ± 4% of the cells to immunostain for pan-keratins and 68 ± 5% to immunostain for E-cadherin, both of which are markers characteristic of epithelial cells (Tseng et al., Cell, 30:361-372 (1982)). Only 4 ± 1% of control cells stained for keratins and 3 ± 2% for E-cadherin. Cells that stained positive for E-cadherin consistently stained for keratins.

Thus, MDSCs of the invention can be induced to differentiate into non-blood cell types, such as epithelial cells, by exposure to effective quantities of a differentiation inducer, such as EGF. One of skill in the art will recognize that other known inducers of epithelial cell differentiation may be substituted for the exemplified inducing compound, EGF. Moreover, skilled artisans will appreciate that suitable dosages of the inducing compounds can be determined using routine techniques well known in the art.

### Example 5

### Neuronal cell differentiation

To examine the ability of MDSCs to mature along yet another cell lineage, the effect of nerve growth factor (bNGF), an inducer of neuronal differentiation (McAllister et al., Cell. Mol. Life Sci., 58:1054-10G0 (2001)), was tested. Four MDSC cultures prepared as described above were treated with 200 ng/ml bNGF, which caused about 90% of the MDSCs to display a neuronal morphology. These cells had a smaller cell body and displayed neurite- and axon-like processes (Jacovina et al., J. Biol. Chem., 276:49350-49358 (2001)). After 5-8 days these processes, some of which were exceedingly long, formed cell-cell contacts and created the appearance of a neural network. These mature cells were further characterized by immunostaining for neuron-specific enolase (NSE), neurofilament (NF) and microtubule-associated protein -1B (MAP-1B), which are well-known markers of neuronal cells (Encinas et al., J. Neurochem., 75:991-1003 (2000)). After three days of treatment, 25% of the cells displayed robust immunostaining for these three proteins and after 5-8 days, this staining was detected in about 90% of the cells, which at this time was also observed in their processes, especially with regards to MAP-1B. After 5-8 days of incubation, less than 9% of control cells displayed elongated processes and these cells stained only weakly for the neuron-specific antigens. Little to no neuronal differentiation was observed when freshly cultured peripheral blood monocytes were treated with bNGF for 7 or 20 days.

Thus, MDSCs of the invention can be induced to differentiate into neuronal cells by exposure to effective quantities ofbNGF. One of skill in the art will recognize that other known inducers of neuronal cell differentiation may be substituted for the exemplified inducing compound, bNGF and, again, skilled artisans will appreciate that suitable dosages of the inducing compounds can be determined using routine techniques well known in the art.

### Example 6

### Endothelial cell differentiation

MDSC cultures prepared as described above were treated with 50 ng/ml of recombinant human vascular endothelial growth factors isoform (VEGF) for 5-7 days. This treatment induced about 70% of the cells to display endothelial cell morphology. A fraction of these cells formed chains of cobblestone-like formations, some of which were parallel or crossed each other. VEGF-treatment also caused 74 ± 3% of the cells to immunostain for three well-known endothelial cell maturation markers (Karkkainen et al., Nature Cell Biol., 4:E2-5 (2002)), namely VEGF-R2, VEGF-R3 and von Willebrand's Factor (vWF). In the absence of VEGF, only 5 ± 1% of the cells stained for these markers. VEGF treatment also induced 31 ± 4% of the cells to immunostain for the neuronal markers NSE, NF and MAP-1B, compared to 7 ± 4% in the absence of VEGF. Nearly all of the NSE-, NF- and MAP-1B-stained cells exhibited a neuronal morphology. A small percentage of cells, which displayed an intermediate morphology between endothelial and neuronal cells, stained for both the endothelial and neuronal markers.

Thus, MDSCs of the invention can be induced to differentiate into endothelial cells by exposure to effective quantities of VEGF. One of skill in the art will recognize that other known inducers of endothelial cell differentiation may be substituted for the exemplified inducing compound, VEGF. Moreover, skilled artisans will appreciate that suitable dosages of the inducing compounds can be determined using routine techniques well known in the art.

### Example 7

### Hepatocyte differentiation

To determine whether MDSCs can also differentiate into liver cells, MDSC cultures prepared as described above were treated for 5-7 days with 100 ng/ml recombinant human hepatocyte growth factor (HGF), a promoter of liver cell growth and differentiation (Michalopoulus and DeFrances, Science 276: 60-66 (1997); Schmidt et al., Nature, 373: 699-702 (1995)). After this treatment, 75-80% of the cells displayed a round or oval-like flattened morphology. It also caused 75±7% of the treated cells to display immunostaining for albumin and 81± 7% to exhibit immunostaining for a fetal protein (AFP) (Table 3), which are specific for differentiated hepatocytes (Hamazaki et al., FEBS Lett. , 497: 15-19 (2001)). A smaller fraction of 33± 4% also immunostained for cytokeratin 7, which is a marker of bile duct epithelium (Ruck et al., Histopathology 31: 324-329 (1997)). Only 8 ± 5% of control cells immunostained for albumin, 6 ± 5% for AFP, and 7 ± 3% for cytokeratin 7.

Thus, MDSCs of the invention can be induced to differentiate into hepatocytes by exposure to effective quantities of HGF. One of skill in the art will recognize that other known inducers of hepatocyte differentiation may be substituted for the exemplified inducing compound, HGF and, again, skilled artisans will appreciate that suitable dosages of the inducing compounds can be determined using routine techniques well known in the art.

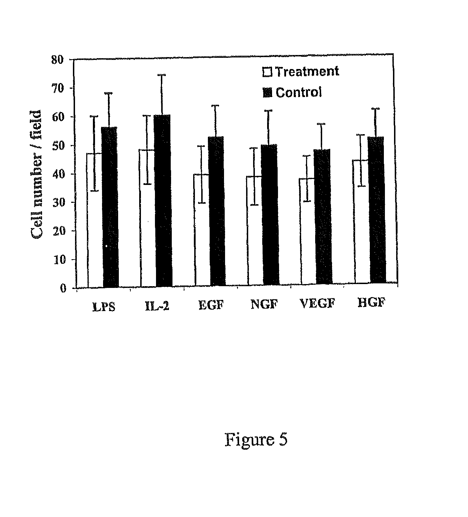

Further, the separate inductions of lymphocytic, epithelial, neuronal, endothelial and hepatocyte cell differentiation from MDSCs, which were associated with a somewhat lower cell number than the control (Fig. 5), were characterized by a marked decrease or disappearance of MAC-1 expression.

The examples herein demonstrate that MDSCs can be induced to differentiate into a variety of cell types from all three germ layers and it is expected that inducers of any of a wide variety of cell type differentiations will be effective with MDSCs.

**Table 3**

| **Treatment** | **Percentage of cells displaying immunostaining for** | | |
|---|---|---|---|
| | **Albumin** | **AFP** | **Cytokeratin 7** |
| **Control** | 8 ± 5 | 6 ± 5 | 7 ± 3 |
| **HGF (50 ng/ml)** | 75 ± 7 | 81 ± 7 | 33 ± 4 |

### Example 8

### Clonal analysis

To determine whether progeny of colonies derived from single MDSCs can be induced to differentiate into distinct cell lineages, cells from a five-day 50 ng/ml M-CSF-treated culture enriched to contain 99.97% peripheral blood monocytes were inoculated into 12 U-bottom tissue culture plates, 96 wells each, at 0.8 cells/well in 0.1-0.2 ml growth medium. The cells were then incubated in the presence of 50 ng/ml M-CSF and 1,000 units/ml LIF. Additionally, one plate was incubated with 25% conditioned medium from a five-day 50 ng/ml M-CSF treated culture. Microscopic inspection indicated that about 70% of the wells contained single cells. The few wells that contained more than one cell were excluded from further experimentation. Medium was replaced every 5-7 days. At 20 days, there were about 5 colonies/plate (about 30 cells/colony). Further incubation caused the cells in most of these colonies to acquire distinct morphologies characteristic of different cell lineages and thereafter to die.

A number of colonies in the plate treated with the conditioned medium continued to grow. At 45-52 days, these cells were dispersed by forceful pipetting, without lidocaine, into flat-bottom, 96-well, tissue culture plates. The untreated cells displayed CD14, CD34 and CD45 cell surface antigens, and most displayed a morphology characteristic of MDSCs. Two colonies of MDSCs, each of which had arisen from a single cell, were chosen for further differentiation experiments and were termed Clone 1 and Clone 2. Seven days after treatment with 1200 units/ml IL-2, 100 ng/ml EGF, 200 ng/ml NGF, 50 ng/ml VEGF or 50 ng/ml HGF, the cells were stained with lineage-specific antibodies. These antibodies separately recognized cell-surface markers that included the T lymphocyte marker CD3, the epithelial marker keratin, the endothelial marker vWF, the neuronal marker MAP1-B, and the hepatocyte marker AFP. The results indicated that the differentiation inducers evoked morphologies consistent with the expected lineages in a majority of the treated cells and were similar to those previously observed for inducer-treated MDSC cultures. As shown in Table 4, 70-90% of the treated cells displayed maturation markers that characterize the specific mature state (Table 4). These observations indicate that progeny of single MDSC have the ability to be induced to differentiate into distinct cell lineages, and as a consequence further confirmed the pluripotent nature of the MDSCs.

Thus, according to methods of the invention, single monocytes can generate an MDSC colony, and the progeny of these can be induced to differentiate into a variety of non-terminally and terminally differentiated cell types. Further, a differentiated cell generated using the methodology disclosed herein can be used in a method for identifying a therapeutic compound, such as a cell type-specific therapeutic compound.

In methods for identifying therapeutic compounds, techniques known in the art are practiced to bring candidate therapeutic compounds into contact with a differentiated cell. In one embodiment, a candidate therapeutic compound is separately brought into contact with a differentiated cell of a first type (e.g., a neuronal cell) and a differentiated cell of a second type (e.g., a macrophage) and measuring the absolute or relative viabilities of the cells. Viability is assessed in terms of any measure acceptable in the art, including a determination of absolute or relative cell number(s), as well as any acceptable measure of the absolute or relative health of a cell (e.g., energy store). Candidate therapeutic compound concentrations are optimized by routine screening using conventional techniques.

Numerous modifications and variations of the invention as set forth in the above illustrative examples are expected to occur to those skilled in the art and are contemplated by the invention. Consequently, only such limitations as appear in the appended claims should be placed on the invention.

**Table 4**

| **Inducer** | **Lineage** | **Marker** | **Treatment** | **Clone 1** | **Clone 2** |
|---|---|---|---|---|---|
| | | | | Immunostained cells (%) | |
| IL-2 | Lymphocyte | CD3 | - | 6 | 3 |
| | | | + | 75 | 81 |
| EGF | Epithelial | Keratins | - | 7 | 5 |
| | | | + | 89 | 76 |
| NGF | Neuronal | MAP1-B | - | 3 | 4 |
| | | | + | 83 | 80 |
| VEGF | Endothelial | vWF | - | 8 | 5 |
| | | | + | 80 | 87 |
| HGF | Hepatocyte | AFP | - | 7 | 2 |
| | | | + | 88 | 75 |

## Claims

1. An isolated monocyte-derived stem cell (MDSC), wherein the cell exhibits a surface antigen selected from the group consisting of MAC-1, CD14, CD34, CD40 and CD45.

2. An isolated monocyte-derived stem cell (MDSC), wherein the cell produces detectable levels of a cytokine selected from the group consisting of interleukin-lp (IL-1β), interleukin-6 (IL-6) and interleukin-12p70 (IL-12p70).

3. An isolated monocyte-derived stem cell (MDSC), wherein the cell exhibits phagocytic activity.

4. An isolated monocyte-derived stem cell (MDSC), wherein the cell is resistant to dispersion by an agent selected from the group consisting of trypsin, EDTA, and dispase.

5. An isolated monocyte-derived stem cell (MDSC), wherein the cell is an adult human cell, further wherein the cell exhibits a surface antigen selected from the group consisting of MAC-1, CD14, CD34, CD40 and CD45, further wherein the cell produces a cytokine selected from the group consisting of interleukin-1β (1L-1β), interleukin-6 (IL-6) and interleukin-12p70 (IL-12p70), further wherein the cell is resistant to dispersion by an agent selected from the group consisting of trypsin, EDTA, and dispase, and further wherein the cell exhibits phagocytic activity.

6. A method of preparing an isolated MDSC, comprising the steps of:
(a) isolating a peripheral-blood monocyte (PBM);
(b) contacting said PBM with an effective amount of a mitogenic compound selected from the group consisting of macrophage colony-stimulating factor (M-CSF), interleukin-6 (IL-6), and leukemia inhibitory factor (LIF); and
(c) culturing said PBM under conditions suitable for propagation of said cell, thereby obtaining a preparation of an isolated MDSC.

7. The method according to claim 6, wherein the PBM is cryopreserved prior to contacting said PBM with a mitogenic compound.

8. The method according to claim 6 or 7, further comprising the cryopreservation of said MDSC.

9. The method according to any of claims 6 to 8, wherein the PBM is a mammalian PBM.

10. The method according to claim 9, wherein the PBM is a human PBM.

11. The method according to claim 10, wherein the PBM is an adult human PBM.

12. An isolated MDSC obtained by the method according to any of claims 6 to 11.

13. A method of generating a differentiated cell, comprising the steps of:
(a) isolating an MDSC by the method according to any of claims 6 to 11;
and
(b) contacting the stem cell with an amount of an inducing agent effective to induce differentiation of the cell, thereby generating a differentiated cell.

14. The method according to claim 13, further comprising cryopreserving said differentiated cell.

15. The method according to claim 13 or 14, further comprising culturing said differentiated cell.

16. The method according to claim 15, wherein the differentiated cell/inducing agent are selected from the group consisting of a neuronal cell/nerve growth factor (bNGF), an endothelial cell/-vascular endothelial growth factor (VEGF), an epithelial cell/epidermal growth factor (EGF), a T-lymphocyte/ interleukin-2 (IL-2), a macrophage/lipopolysaccharide (LPS), and a hepatocyte/hepatocyte growth factor (HGF).

17. The method according to any of claims 13 to 16, wherein the MDSC is a human MDSC.

18. The method according to claim 17, wherein the MDSC is an adult human MDSC.

19. A method for identifying a cell type-specific therapeutic agent, comprising:
(a) contacting a first differentiated cell obtained according to the method of any of claims 13 to 18 and a candidate therapeutic agent;
(b) further contacting a second differentiated cell obtained according to the method of any of claims 13 to 18 and the candidate therapeutic agent, wherein the first and second differentiated cells are different cell types; and
(c) measuring the viability of the first differentiated cell relative to the viability of the second differentiated cell, wherein a difference in viabilities identifies the candidate therapeutic agent as a cell type-specific therapeutic agent.

20. The use of an MDSC for the preparation of a pharmaceutical composition for the treatment of a disorder amenable to cell-based treatment, wherein the MDSC is isolated from the organism to receive treatment.

21. The use of the MDSC according to claim 20, wherein the organism is a human.

22. The use of a neuronal cell obtained by the method according to any of claims 16 to 18 for the preparation of a pharmaceutical composition for the treatment of a neuronal cell disorder amenable to cell-based treatment.

23. The use of an endothelial cell obtained by the method according to any of claims 16 to 18 for the preparation of a pharmaceutical composition for the treatment of an endothelial cell disorder amenable to cell-based treatment.

24. The use of an epithelial cell obtained by the method according to any of claims 16 to 18 for the preparation of a pharmaceutical composition for the treatment of an epithelial cell disorder amenable to cell-based treatment.

25. The use of a T-lymphocyte obtained by the method according to any of claims 16 to 18 for the preparation of a pharmaceutical composition for the treatment of a T-lymphocyte disorder amenable to cell-based treatment.

26. The use of a macrophage obtained by the method according to any of claims 16 to 18 for the preparation of a pharmaceutical composition for the treatment of a macrophage cell disorder amenable to cell-based treatment.

27. The use of a hepatocyte obtained by the method according to any of claims 16 to 18 for the preparation of a pharmaceutical composition for the treatment of a hepatocyte disorder amenable to cell-based treatment.

28. The use of a neuronal cell obtained by the method according to any of claims 16 to 18 for the preparation of a pharmaceutical composition for the treatment of a symptom associated with a neuronal cell disorder amenable to cell-based treatment.

29. The use of an endothelial cell obtained by the method according to any of claims 16 to 18 for the preparation of a pharmaceutical composition for the treatment of a symptom associated with an endothelial cell disorder amenable to cell-based treatment.

30. The use of an epithelial cell obtained by the method according to any of claims 16 to 18 for the preparation of a pharmaceutical composition for the treatment of a symptom associated with an epithelial cell disorder amenable to cell-based treatment.

31. The use of a T-lymphocyte obtained by the method according to any of claims 16 to 18 for the preparation of a pharmaceutical composition for the treatment of a symptom associated with a T-lymphocyte disorder amenable to cell-based treatment.

32. The use of a macrophage obtained by the method according to any of claims 16 to 18 for the preparation of a pharmaceutical composition for the treatment of a symptom associated with a macrophage cell disorder amenable to cell-based treatment.

33. The use of a hepatocyte obtained by the method according to any of claims 16 to 18 for the preparation of a pharmaceutical composition for the treatment of a symptom associated with a hepatocyte disorder amenable to cell-based treatment.

34. A pharmaceutical composition comprising an MDSC and a pharmaceutically acceptable diluent, carrier or medium.

35. A kit comprising the pharmaceutical composition according to claim 34.
